# EUROPEAN PATENT SPECIFICATION

(11) **EP 2 759 269 B1**
(45) Date of publication and mention of the grant of the patent: **30.03.2016**
(21) Application number: 14152390.2
(22) Date of filing: 24.01.2014
(51) Int. Cl.: A61B 17/072

(54) **Foam application to stapling device**
Schaumauftragung auf Heftvorrichtung
Dispositif d'agrafage avec application de mousse

(30) Priority: 25.01.2013 US 201361756627 P; 19.12.2013 US 201314134636
(43) Date of publication of application: 30.07.2014
(73) Proprietor: Covidien LP, Mansfield, MA 02048 (US)
(72) Inventor: Skalla, Walter, Old Lyme, CT Connecticut 06371 (US); Bennett, Steven L., Cheshire, CT Connecticut 06410 (US); Racenet, Danyel, Killingworth, CT Connecticut 06419 (US)
(74) Representative: Soames, Candida Jane

(56) References cited:
- EP-A1- 1 064 883
- EP-A2- 2 008 595
- EP-A2- 2 604 194
- US-A- 5 104 025
- US-A- 5 397 324
- US-A1- 2002 165 562

## Description

### CROSS-REFERENCE TO RELATED APPLICATIONS

The present application claims priority to, and the benefit of, U.S. Provisional Patent Application Serial No. 61/756,627, filed on January 25, 2013.

### BACKGROUND

### Technical Field

The present disclosure relates to a surgical stapling apparatus including a surgical buttress attached to a staple cartridge and/or an anvil assembly of the surgical stapling apparatus with an adhesive, and more particularly, to a hydrophilic buttress releasably attached to a surgical stapling apparatus with a low molecular weight biodegradable polymer.

### Background of Related Art

Surgical stapling apparatuses are employed by surgeons to sequentially or simultaneously apply one or more rows of fasteners, e.g., staples or two-part fasteners, to body tissue for the purpose of joining segments of body tissue together. Such apparatuses generally include a pair of jaws or finger-like structures between which the body tissue to be joined is placed. When a stapling apparatus is actuated, or "fired", longitudinally moving firing bars contact staple drive members in one of the jaws. The staple drive members push the staples through the body tissue and into an anvil in the opposite jaw which forms the staples. If tissue is to be removed or separated, a knife blade can be provided in the jaws of the apparatus to cut the tissue between the staples.

Buttresses may be used with surgical stapling apparatuses to reinforce the staple line and reduce the incidence of leaks and bleeding. A number of surgical stapling apparatuses, however, rely on knife blade cutting of some portion of the buttress to affect release of the buttress from the surgical stapling apparatus, or the use of secondary materials or mounting structures to attach the buttress to the surgical stapling apparatus. Drawbacks to these approaches include unreliable detachment of the buttress from the surgical apparatus as they may require increased firing forces to transect the buttress by the knife blade to release the buttress and/or the need to remove excess or secondary material when the surgical stapling apparatus is withdrawn.

It would be desirable to provide a buttress that is reliably retained on a surgical stapling apparatus without the use of excess material that can be detached from the surgical stapling apparatus when the apparatus is removed from the surgical site without the need for knife blade cutting.

Various types of surgical buttresses are known from EP 1064883 which discloses the preamble of claim 1, US 2002/165562, 2604194, EP 2008595 and US 5397324.

### SUMMARY

According to an aspect of the present disclosure, a surgical stapling apparatus includes a cartridge assembly, an anvil assembly, a surgical buttress, and a low molecular weight biodegradable adhesive. The cartridge assembly includes a plurality of staples and a tissue contacting surface defining staple retaining slots. The anvil assembly includes a tissue contacting surface defining staple pockets for forming staples expelled from the staple retaining slots of the cartridge assembly. The surgical buttress is disposed on at least one of the tissue contacting surfaces of the cartridge assembly and the anvil assembly. The low molecular weight biodegradable adhesive releasably retains the surgical buttress on at least one of the tissue contacting surfaces of the cartridge assembly and the anvil assembly.

In embodiments, of the invention the surgical buttress is porous. The surgical buttress includes a foam layer. A non-woven fabric layer is attached to a surface of the foam layer. The non-woven layer is a non-woven fabric layer and adhered to at least one of the tissue contacting surfaces of the cartridge assembly and the anvil assembly.

The surgical stapling apparatus may include a knife disposed within a knife slot formed in the tissue contacting surface of the cartridge assembly. In such embodiments, the surgical buttress may include a gap to allow free passage of the knife through the knife slot.

The low molecular weight biodegradable adhesive has a molecular weight that is less than 3,000 g/mol. In embodiments, the molecular weight of the low molecular weight biodegradable adhesive may be from about 1,000 g/mol to about 2,300 g/mol. In embodiments, the molecular weight of the low molecular weight biodegradable adhesive may be from about 1,300 g/mol to about 2,000 g/mol.

According to another aspect of the present disclosure, a surgical stapling apparatus includes a cartridge assembly, an anvil assembly, a surgical buttress, and a water dissolvable adhesive. The cartridge assembly includes a plurality of staples and a tissue contacting surface defining staple retaining slots. The anvil assembly includes a tissue contacting surface defining staple pockets for forming staples expelled from the staple retaining slots of the cartridge assembly. The surgical buttress is disposed on at least one of the tissue contacting surfaces of the cartridge assembly and the anvil assembly. The water dissolvable adhesive releasably retains the surgical buttress on the at least one of the tissue contacting surfaces of the cartridge assembly and the anvil assembly.

### BRIEF DESCRIPTION OF THE DRAWINGS

Embodiments of the presently disclosed surgical stapling apparatus and surgical buttress are described herein with reference to the accompanying drawings, wherein:
FIG. 1 is a perspective view of an illustrative example of the present disclosure of a surgical stapling apparatus including a surgical buttress disposed on a staple cartridge of the surgical stapling apparatus and a surgical buttress disposed on an anvil assembly of the surgical stapling apparatus;
FIG. 2A is a schematic side illustration of a surgical buttress in accordance with an embodiment of the present invention ;
FIGS. 2B and 2C are schematic illustrations of the distribution patterns of an adhesive on surgical buttresses in accordance with embodiments of the present disclosure;
FIG. 3 is a perspective view of a distal end of the surgical stapling apparatus of FIG. 1, shown in use positioned about a tissue section;
FIG. 4 is a cross-sectional view taken along line 4-4 of FIG. 3;
FIG. 5 is a perspective view of the stapled and divided tissue section of FIG. 4;
FIG. 6A is a perspective view of an illustrative example of a surgical stapling apparatus in accordance with the present disclosure;
FIG. 6B is a cross-sectional view of the surgical stapling apparatus of FIG. 6A including a surgical buttress positioned within an intestinal area; and
FIG. 6C is a top view of the surgical buttress of FIG. 6B.

### DETAILED DESCRIPTION OF THE EMBODIMENTS

Various exemplary embodiments of the present disclosure are discussed herein below in terms of buttresses for use with a surgical stapling apparatus, and adhesives for attaching the buttresses to the surgical stapling apparatus. The buttresses described herein may be used to seal a wound by approximating the edges of wound tissue between a staple cartridge assembly and an anvil assembly of a surgical stapling apparatus, and to absorb any blood present at the wound tissue site. The buttress may be releasably joined to the surgical stapling apparatus by a low molecular weight biodegradable polymer. Thus, the present disclosure describes surgical buttresses and adhesives for securing the surgical buttresses to surgical stapling apparatuses, and methods for making and using the same.

It should be understood that a variety of surgical stapling apparatuses may be utilized with a surgical buttress and an adhesive of the present disclosure. For example, linear staplers may be utilized such as, for example, those including Duet TRS™ reloads and staplers with Tri-Staple™ technology, available through Covidien, (North Haven, CT), as well as other anastomosis staplers, such as, for example, EEA™, CEEA™, GIA™, EndoGIA™, and TA™, also available through Covidien. It should also be appreciated that the principles of the present disclosure are equally applicable to surgical staplers having alternate configurations, such as, for example, end-to-end anastomosis staplers having a circular cartridge and anvil (see, e.g., commonly owned U.S. Patent No. 5,915,616, entitled "Surgical Fastener Applying Apparatus," ); laparoscopic staplers (see, e.g., commonly owned U.S. Pat. Nos. 6,330,965 and 6,241,139 , each entitled "Surgical Stapling Apparatus"); and transverse anastomosis staplers (see, e.g., commonly owned U.S. Patent Nos. 5,964,394 and 7,334,717 , each entitled "Surgical Fastener Applying Apparatus").

Embodiments of the presently disclosed surgical buttress, adhesive, and surgical stapling apparatus will now be described in detail with reference to the drawing figures wherein like reference numerals identify similar or identical elements. In the following discussion, the terms "proximal" and "trailing" may be employed interchangeably, and should be understood as referring to the portion of a structure that is closer to a clinician during proper use. The terms "distal" and "leading" may also be employed interchangeably, and should be understood as referring to the portion of a structure that is further from the clinician during proper use. As used herein, the term "patient" should be understood as referring to a human subject or other animal, and the term "clinician" should be understood as referring to a doctor, nurse, or other care provider and may include support personnel.

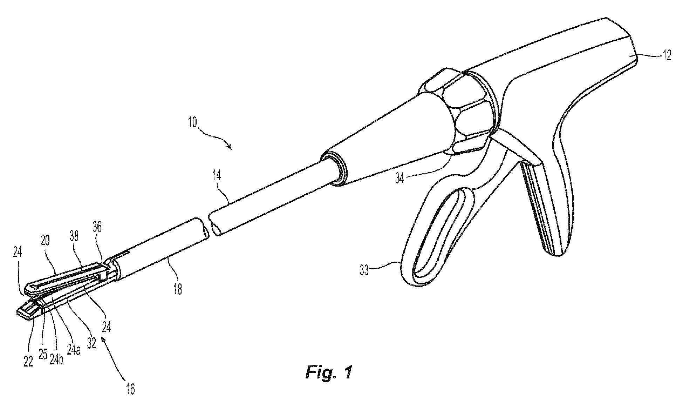

Referring now to FIG. 1, there is disclosed an exemplary surgical stapling apparatus or surgical stapler 10 for use in stapling tissue and applying a buttress material or surgical buttress to tissue. Surgical stapling apparatus 10 generally includes a handle 12 having an elongate tubular member 14 extending distally from handle 12. A jaw assembly 16 is mounted on a distal end 18 of elongate tubular member 14. Jaw assembly 16 includes an anvil assembly including a staple clinching anvil jaw member 20 and a cartridge assembly including a receiving jaw member 22 configured to receive a staple cartridge 32. Jaw assembly 16 may be permanently affixed to elongate tubular member 14 or may be detachable and thus replaceable with a new jaw assembly 16. Staple clinching anvil jaw member 20 is movably mounted on distal end 18 of jaw assembly 16 and is movable between an open position spaced apart from staple cartridge jaw member 22 to a closed position substantially adjacent staple cartridge jaw member 22.

Surgical stapling apparatus 10 further includes a trigger 33, as seen in FIG. 1, movably mounted on handle 12. Actuation of trigger 33 initially operates to move anvil jaw member 20 from the open to the closed position relative to staple cartridge jaw member 22 and subsequently actuates surgical stapling apparatus 10 to apply lines of staples to tissue. In order to properly orient jaw assembly 16 relative to the tissue to be stapled, surgical stapling apparatus 10 is additionally provided with a rotation knob 34 mounted on handle 12. Rotation of rotation knob 34 relative to handle 12 rotates elongate tubular member 14 and jaw assembly 16 relative to handle 12 so as to properly orient jaw assembly 16 relative to the tissue to be stapled.

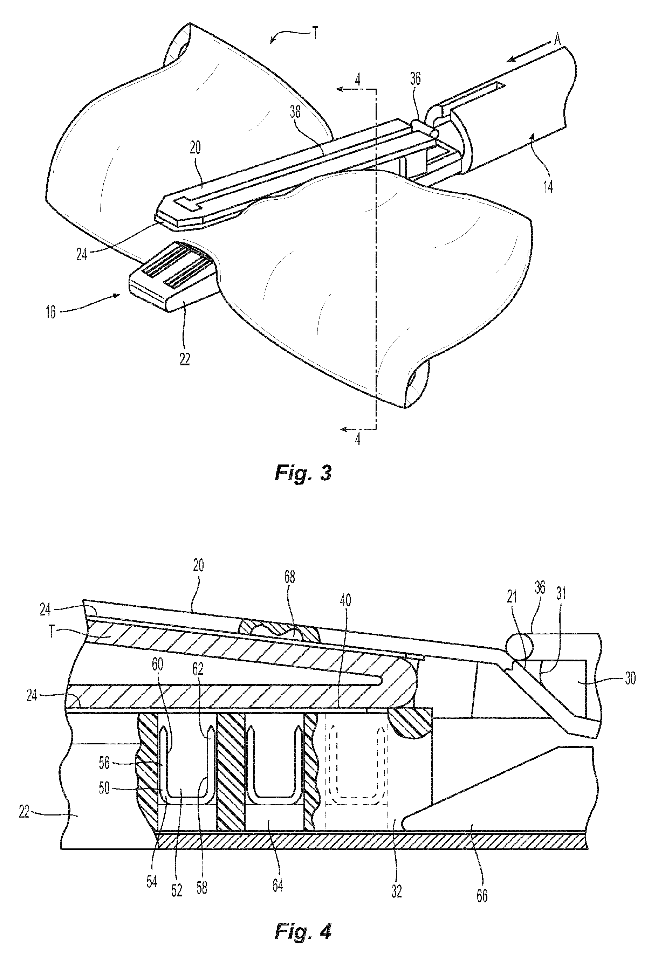

A driver 36, as seen in FIGS. 3 and 4, is provided to move anvil jaw member 20 between the open and closed positions relative to staple cartridge jaw member 22. Driver 36 moves between a longitudinal slot 38 formed in anvil jaw member 20. A knife 30, disposed within knife slot 25 (FIG. 1), is associated with driver 36 to cut tissue captured between anvil jaw member 20 and staple cartridge jaw member 22 as driver 36 passes through slot 38.

Reference may be made to commonly owned U.S. Patent Nos. 5,915,616, 6,330,965, and 6,241,139, referenced above, for a detailed discussion of the construction and operation of surgical stapling apparatus 10.

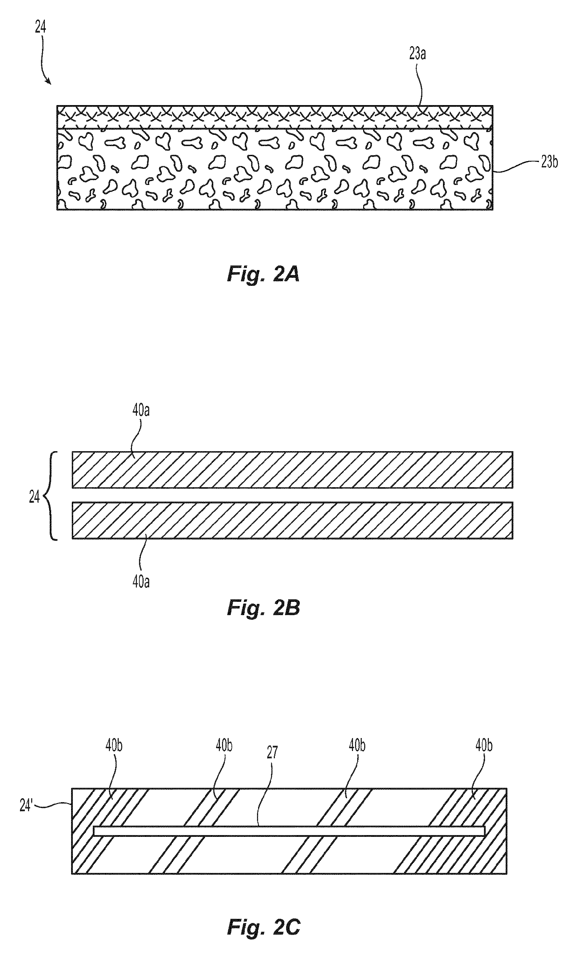

Referring again to FIG. 1, staple clinching anvil jaw member 20 and/or staple cartridge jaw member 22 may be provided with a surgical buttress 24. Surgical buttress 24 is provided to reinforce and seal staple lines applied to tissue by surgical stapling apparatus 10. Surgical buttress 24 is illustrated as including two separate pieces 24a and 24b that are placed on each side of knife slot 25. However, surgical buttress 24' may be fabricated as a single piece that includes a gap 27 therein to allow free passage of the knife through the knife slot, as illustrated in FIG. 2C. Surgical buttress 24 may be configured in any shape, size, or dimension suitable to fit any surgical stapling, fastening, or firing apparatus.

Surgical buttress 24 is fabricated from a biocompatible material which can be any suitable bioabsorbable and/or biodegradable, non-absorbable, natural and/or synthetic material. It should of course be understood that any combination of natural, synthetic, bioabsorbable, biodegradable, and non-bioabsorbable materials may be used to form the surgical buttress.

Representative natural biodegradable polymers from which the surgical buttress may be formed include: polysaccharides such as alginate, dextran, chitin, chitosan, hyaluronic acid, cellulose, collagen, gelatin, fucans, glycosaminoglycans, and chemical derivatives thereof (substitutions and/or additions of chemical groups include, for example, alkyl, alkylene, amine, sulfate, hydroxylations, carboxylations, oxidations, and other modifications routinely made by those skilled in the art); catgut; silk; linen; cotton; and proteins such as albumin, casein, zein, silk, soybean protein, and copolymers and blends thereof; alone or in combination with synthetic polymers.

Synthetically modified natural polymers which may be used to form a buttress include cellulose derivatives such as alkyl celluloses, hydroxyalkyl celluloses, cellulose ethers, cellulose esters, nitrocelluloses, and chitosan. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate, and cellulose sulfate sodium salt.

Representative synthetic biodegradable polymers used to form a buttress include polyhydroxy acids such as glycolide, lactide, caprolactone, ε-caprolactone, valerolactone, and δ-valerolactone, carbonates (e.g., trimethylene carbonate, tetramethylene carbonate, and the like), dioxanones (e.g., 1,4-dioxanone and p-dioxanone), dioxepanones (e.g., 1,4-dioxepan-2-one and 1,5-dioxepan-2-one), and combinations thereof. Polymers formed therefrom include: polylactides; poly(lactic acid); polyglycolides; poly(glycolic acid); poly(trimethylene carbonate); poly(dioxanone); poly(hydroxybutyric acid); poly(hydroxyvaleric acid); poly(lactide-co-(ε-caprolactone-)); poly(glycolide-co-(ε-caprolactone)); polycarbonates; poly(pseudo amino acids); poly(amino acids); poly(hydroxyalkanoate)s such as polyhydroxybutyrate, polyhydroxyvalerate, poly(3-hydroxybutyrate-co-3-hydroxyvalerate), polyhydroxyoctanoate, and polyhydroxyhexanoate; polyalkylene oxalates; polyoxaesters; polyanhydrides; polyester anhydrides; polyortho esters; and copolymers, block copolymers, homopolymers, blends, and combinations thereof.

Some non-limiting examples of suitable non-degradable materials used to form a buttress include: polyolefins such as polyethylene (including ultra high molecular weight polyethylene) and polypropylene including atactic, isotactic, syndiotactic, and blends thereof; polyethylene glycols; polyethylene oxides; polyisobutylene and ethylene-alpha olefin copolymers; fluorinated polyolefins such as fluoroethylenes, fluoropropylenes, fluoroPEGSs, and polytetrafluoroethylene; polyamides such as nylon, Nylon 6, Nylon 6,6, Nylon 6,10, Nylon 11, Nylon 12, and polycaprolactam; polyamines; polyimines; polyesters such as polyethylene terephthalate, polyethylene naphthalate, polytrimethylene terephthalate, and polybutylene terephthalate; polyethers; polybutester; polytetramethylene ether glycol; 1,4-butanediol; polyurethanes; acrylic polymers; methacrylics; vinyl halide polymers such as polyvinyl chloride; polyvinyl alcohols; polyvinyl ethers such as polyvinyl methyl ether; polyvinylidene halides such as polyvinylidene fluoride and polyvinylidene chloride; polychlorofluoroethylene; polyacrylonitrile; polyaryletherketones; polyvinyl ketones; polyvinyl aromatics such as polystyrene; polyvinyl esters such as polyvinyl acetate; etheylene-methyl methacrylate copolymers; acrylonitrile-styrene copolymers; acrylonitrile butadiene styrene (ABS) resins; ethylene-vinyl acetate copolymers; alkyd resins; polycarbonates; polyoxymethylenes; polyphosphazines; polyimides; epoxy resins; aramids; rayon; rayon-triacetate; spandex; silicones; and copolymers and combinations thereof.

The surgical buttress 24 may be porous, non-porous, or combinations thereof. It is envisioned that surgical buttress 24 described herein may contain a plurality of layers in which any combination of non-porous and porous layers may be configured. It is further envisioned that non-porous and/or porous layers may be positioned in any order relative to the tissue contacting surfaces of the staple cartridge jaw member and/or the anvil jaw member.

The use of non-porous layer(s) in the surgical buttress may enhance the ability of the surgical buttress to resist tears and perforations during the manufacturing, shipping, handling, and stapling processes. Also, the use of a non-porous layer in the surgical buttress may also retard or prevent tissue ingrowth from surrounding tissues, thereby acting as an adhesion barrier and preventing the formation of unwanted scar tissue. A non-porous layer of the surgical buttress may be formed using techniques within the purview of those skilled in the art, such as casting, molding, and the like.

The use of porous layer(s) may enhance the ability of the surgical buttress to absorb fluid, reduce bleeding, and seal the wound. Also, the porous layer(s) may allow for tissue ingrowth to fix the surgical buttress in place. A porous layer may have openings or pores over at least a portion of a surface thereof. As described in more detail below, suitable materials for forming a porous layer include, but are not limited to, fibrous structures (e.g., knitted structures, woven structures, non-woven structures, etc.) and/or foams (e.g., open or closed cell foams).

In embodiments, the pores may be in sufficient number and size so as to interconnect across the entire thickness of the porous layer. Woven fabrics, knitted fabrics, and open cell foam are illustrative examples of structures in which the pores can be in sufficient number and size so as to interconnect across the entire thickness of the porous layer. In embodiments, the pores may not interconnect across the entire thickness of the porous layer, but rather may be present at a portion thereof. Closed cell foam or fused non-woven materials are illustrative examples of structures in which the pores may not interconnect across the entire thickness of the porous layer. Thus, in some embodiments, pores may be located on a portion of the porous layer, with other portions of the porous layer having a non-porous texture. Those skilled in the art reading the present disclosure will envision a variety of pore distribution patterns and configurations for the porous layer.

Where a porous layer of the surgical buttress is fibrous, the fibers may be filaments or threads suitable for knitting or weaving, or may be staple fibers, such as those frequently used for preparing non-woven materials. Suitable techniques for making fibrous structures are within the purview of those skilled in the art.

Where a porous layer of the surgical buttress is a foam, the porous layer may be formed using any method suitable for forming a foam or sponge including, but not limited to, the lyophilization or freeze-drying of a composition. Suitable techniques for making foams are within the purview of those skilled in the art.

In embodiments, a surgical buttress includes a porous foam layer formed of collagen. Collagen may be of human and/or animal origin, e.g., type I porcine or bovine collagen, type I human collagen or type III human collagen, or mixtures in any proportions, including those that are chemically modified by oxidation, methylation, succinylation, ethylation, or any other known process.

The foam layer may be formed from native collagen (CPP). CPP is without telopeptide, has its helicoidal structure preserved, and has an average molecular weight of about 300,000 g/mol. CPP may be prepared into a foam via lyophilization. In an exemplary embodiment of making a foam layer of the present disclosure, CPP may be dissolved in water that has been purified by reverse osmosis with gentle mixing and light heat at approximately 45°C. The degradation rate and stiffness of CPP can be varied by its initial concentration in water. In embodiments, the solution may contain less than about 5% w/v CPP, in some embodiments, about 1% w/v CPP. The solution may then be poured into a dish, such as a Teflon dish. It should be understood that foam thickness will vary depending on the volume added to the dish. The solution is then put through a lyophilization cycle. The resulting foam may then be trimmed to a desired length/dimension.

According to the invention the surgical buttress 24 includes a non-woven fabric layer 23a and a foam layer 23b, as illustrated in FIG. 2A. For example, with regard to the embodiment above for making a CPP foam layer, a non-woven fabric may be placed on top of the solution prior to running the dish through the lyophilization cycle. The freeze drying process attaches the non-woven fabric to the foam.

In embodiments, the non-woven layer is formed from filaments of aliphatic polyester. In some embodiments, the non-woven fabric layer may be fabricated from a lactomer copolymer of glycolide and lactide derived from glycolic and lactic acids. In other embodiments, the non-woven fabric layer may be fabricated from polyglyconate, a copolymer of glycolic acid and trimethylene carbonate. In yet other embodiments, the non-woven fabric layer may be fabricated from a synthetic polyester composed of glycolide, dioxanone, and trimethylene carbonate. The polymer may include from about 50% to about 70% by weight glycolide, in embodiments, from about 55% to about 65% by weight glycolide, and in some embodiments, about 60% by weight glycolide; from about 4% to about 24% by weight dioxanone, in embodiments, from about 9% to about 19% by weight dioxanone, and in some embodiments, about 14% by weight dioxanone; and from about 16% to about 36% by weight trimethylene carbonate, in embodiments, from about 21% to about 31% by weight trimethylene carbonate, and in some embodiments, about 26% by weight trimethylene carbonate.

The surgical buttress is attached to the surgical stapling apparatus with a biodegradable adhesive. The biodegradable adhesive may be fabricated from biocompatible natural or synthetic polymers. Examples of suitable polymers include, but are not limited to, aliphatic polyesters; polyamides; polyamines; polyalkylene oxalates; poly(anhydrides); polyamidoesters; copoly(ether-esters); poly(carbonates); poly(hydroxyalkanoates); polyimide carbonates; poly(imino carbonates); polyorthoesters; polyoxaesters; polyphosphazenes; poly (propylene fumarates); polyurethanes; polymer drugs; biologically modified (e.g., protein, peptide) bioabsorbable polymers; and copolymers, block copolymers, homopolymers, blends, and combinations thereof.

In embodiments, aliphatic polyesters are utilized as the adhesive. Suitable aliphatic polyesters include, but are not limited to, homopolymers and copolymers of lactide (including lactic acid, D-,L- and meso lactide); glycolide (including glycolic acid); epsilon-caprolactone; p-dioxanone (1,4-dioxan-2-one); trimethylene carbonate (1,3-dioxan-2-one); alkyl derivatives of trimethylene carbonate; Δ-valerolactone; β-butyrolactone; γ-butyrolactone; ε-decalactone; hydroxybutyrate; hydroxyvalerate; 1,4-dioxepan-2-one; 1,5-dioxepan-2-one; 6,6-dimethyl-1,4-dioxan-2-one; 2,5-diketomorpholine; pivalolactone; α,α diethylpropiolactone; ethylene carbonate; ethylene oxalate; 3-methyl-1,4-dioxane-2,5-dione; 3,3-diethyl-1,4-dioxan-2,5-dione; 6,8-dioxabicycloctane-7-one; and polymer blends and combinations thereof.

According to the invention the biodegradable adhesive has a low molecular weight of less than 3,000 g/mol. In embodiments, the biodegradable adhesive has a molecular weight from about 1,000 g/mol to about 2,300 g/mol, and in some embodiments, from about 1,300 g/mol to about 2,000 g/mol.

A low molecular weight biodegradable adhesive may be an aliphatic star polymer having multiple arms radiating from a single core. In embodiments a low molecular weight biodegradable aliphatic star polymer may include various monomers, such as, for example, lactone monomers of the aliphatic polyesters described above, which may be polymerized with initiators in a catalyzed reaction. Examples of suitable initiators include, but are not limited to:
diols, such as, ethylene glycol, diethylene glycol, 1,3-propanediol, 1,4-butanediol, 1,5-pentanediol, 1,6-hexanediol, 1,7-heptanediol, 1,8-octanediol, 1,10-decanediol, 1,12-dodecanediol, 1,2-decanediol, 1,2-dodecanediol, 1,2-hexadecanediol, neopentyl glycol, 3-methyl-1,5-pentanediol, 2-methyl-1,3-propanediol, 2-butyl-2-ethyl-1,3-propanediol, 2-ethyl-3-butyl- 1,3-propanediol, 2-ethyl-1,6-hexanediol; aromatic and alkyl triols, such as, for example, glycerol and 1 ,1 ,1- trimethylolpropane; polyols, such as neopentyl glycol, and pentaerythritol; alcohol amines, such as triethanolamine, 1-, and 2-aminopropanols, 2- and 4- aminobutanols and the like; dicarboxylic acids such as succinic acid, glutaric acid, adipic acid, suberic acid, sebacic acid, dodecanedioic acid, and 2-ethyl-2-methylsuccinic acid; and aromatic dicarboxylic acids, such as phthalic acid, isophthalic acid, and terephthalic acid. Examples of suitable catalysts which may be used in forming a star polymer include, for example, organometallic catalysts, such as stannous octoate, stannous chloride, diethyl zinc, and zirconium acetylactetonate.

Conditions for polymerizing monomers in the presence of an initiator are within the purview or those skilled in the art. For example, the biodegradable adhesive can be prepared by drying purified monomer(s) used to form the biodegradable adhesive and then polymerizing the monomers at temperatures from about 20°C to about 220°C, in embodiments, above 75°C, in the presence of an organometallic catalyst. The polymerization time may be from about 1 to about 100 hours or longer, depending on the other polymerization parameters, but generally polymerization times from about 12 to about 48 hours are employed. In addition, as noted above, a multifunctional initiator may be employed. Generally, the amount of initiator used will be from about 0.01 to about 30 percent by weight based on the weight of the monomer(s). In embodiments, the initiator will be present in the reaction mixture in an amount from about 0.5 to about 20 weight percent based on the weight of the monomer(s).

As an alternative example not forming part of the present invention, the biodegradable adhesive may be a water soluble adhesive. In embodiments, the adhesive may be fabricated from water soluble polysaccharides, such as those described above with respect to the surgical buttress, as well as other polysaccharides such as, for example, mannitol, pullalan, and pectin. In embodiments, pullulan may be utilized. Pullulan may have a molecular weight of about 200,000 g/mol. In such embodiments, the biodegradable adhesive may be a water soluble concentrated pullulan solution including approximately 30% w/v pullulan in water that has been purified by reverse osmosis.

The surgical buttress may be attached to the anvil assembly and/or cartridge assembly by warming the biodegradable adhesive and applying it either to the surgical buttress, the tissue facing surface of the anvil assembly and/or the cartridge assembly, or any combination thereof. In embodiments in which the surgical buttress includes a foam layer and a fabric layer, the biodegradable adhesive may be applied to the fabric layer, or to the tissue facing surface of the anvil assembly and/or cartridge assembly, with the fabric layer positioned to face the tissue facing surface. As illustrated in the embodiment shown in FIG. 1, a surgical buttress 24 is releasable attached to both staple cartridge 32 and anvil jaw member 20 by a biodegradable adhesive (not shown) leaving knife slot 25 free for passage of knife 30 therethrough. It should be understood that a surgical buttress may be associated with only the anvil jaw member or the staple cartridge.

The adhesive may be applied as a continuous or discontinuous pattern on the buttress, staple cartridge, and/or anvil jaw member. A continuous coating of biodegradable adhesive 40a on surgical buttress 24 is illustrated in FIG. 2B, and a discontinuous coating of biodegradable adhesive 40b on a surgical buttress 24' is illustrated in FIG. 2C. The pattern may be a systematic or random distribution. It is envisioned that in a discontinuous adhesive pattern, the amount of adhesive, size, and spacing may be varied to optimize the attachment of the surgical buttress to the surgical stapling apparatus, as well as to minimize the detachment force required during firing.

The adhesive is releasably attached to the staple cartridge and/or the anvil jaw member in a manner which allows the surgical buttress to be removed or released from the staple cartridge and/or the anvil jaw member by the staples upon firing of the surgical stapling apparatus. The adhesive is strong enough to adhere the surgical buttress to the staple cartridge and/or anvil assembly during placement within tissue, yet will soften, and/or degrade upon exposure to body fluids, such as blood, and release the surgical buttress when the surgical buttress is impacted or penetrated by the staples.

As illustrated in FIG. 3, during use of surgical stapling apparatus 10, the anvil jaw member 20 and the staple cartridge jaw member 22 including a staple cartridge (not shown), which have each been loaded with a surgical buttress 24 by a biodegradable adhesive (not shown), are positioned on both sides of the surgical site where adjacent layers of tissue "T" are to be fastened to one another.

As best shown in FIG. 4, staple cartridge 32 includes surgical staples 50 positioned within individual staple pockets 52. Staples 50 are of a conventional type and include a backspan 54 having a pair of legs 56 and 58 extending from backspan 54. Legs 56 and 58 terminate in tissue penetrating tips 60 and 62, respectively. Pushers 64 are located within staple pockets 52 and are positioned between staples 50 and the path of a drive bar 66.

Surgical stapling apparatus 10 is initially actuated by movement of trigger 33 relative to handle 12 (FIG. 1) causing driver 36 to move in the direction of arrow "A" (FIG. 3), and against sloped edge 21 of anvil jaw member 20 thereby causing anvil jaw member 20 to be moved to the closed position relative to staple cartridge jaw member 22. As drive bar 66 advances distally within staple cartridge 32, drive bar 66 urges pushers 64 upwardly against backspan 54 of staples 50 driving legs 56 and 58 of staples 50 through surgical buttress 24 associated with the staple cartridge jaw member 22, tissue "T", surgical buttress 24 associated with anvil jaw member 20, and towards staple forming pockets 68 in anvil jaw member 20. Tissue penetrating tips 60 and 62 of staple legs 56 and 58 are bent within staple forming pockets 68 in anvil jaw member 20 with backspan 54 securing surgical buttress 24 against tissue "T".

Upon full actuation of surgical stapling apparatus 10, blade 31 of knife 30, which is carried by driver 36, cuts the tissue "T" between the rows of now formed staples 50. Upon movement of anvil jaw member 20 to the open position spaced apart from staple cartridge jaw member 22, surgical buttresses 24 are pulled away from anvil jaw member 20 and staple cartridge 32 of staple cartridge jaw member 22.

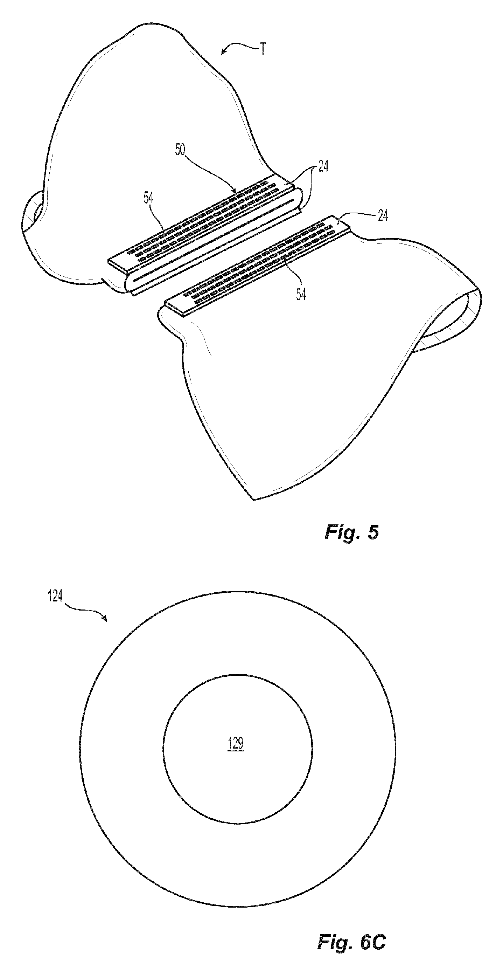

The resulting tissue "T", divided and stapled closed with staples 50, is illustrated in FIG. 5. Specifically, the surgical buttress 24 that was associated with the staple cartridge jaw member 22 is secured against tissue "T" by backspans 54 of staples 50 and the surgical buttress 24 associated with the anvil jaw member 20 is secured against tissue "T" by staple legs 56 and 58. Thus, surgical buttresses 24 are stapled to tissue "T" thereby sealing and reinforcing the staple lines created by staples 50.

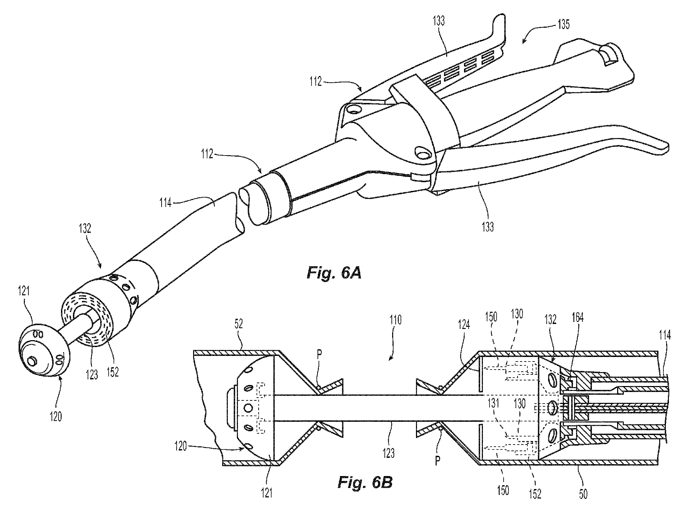

Referring now to FIGS. 6A and 6B, an annular surgical stapling apparatus 110, for use with a surgical buttress 124 of the present disclosure, is shown. Surgical stapling apparatus 110 includes a handle assembly 112 having at least one pivotable actuating handle member 133, and an advancing member 135. Extending from handle member 112, there is provided a tubular body portion 114 which may be constructed so as to have a curved shape along its length. Body portion 114 terminates in a staple cartridge assembly 132 which includes a pair of annular arrays of staple receiving slots 152 having a staple 150 disposed in each one of staple receiving slots 152. Positioned distally of staple cartridge assembly 132 there is provided an anvil assembly 120 including an anvil member 121 and a shaft 123 operatively associated therewith for removably connecting anvil assembly 120 to a distal end portion of stapling apparatus 110.

Staple cartridge assembly 132 may be fixedly connected to the distal end of tubular body portion 114 or may be configured to concentrically fit within the distal end of tubular body portion 114. Typically, staple cartridge assembly 132 includes a staple pusher 164 including a proximal portion having a generally frusto-conical shape and a distal portion defining two concentric rings of peripherally spaced fingers (not shown), each one of which is received within a respective staple receiving slot 152.

A knife 130, substantially in the form of an open cup with the rim thereof defining a knife blade 131, is disposed within staple cartridge assembly 132 and mounted to a distal surface of a staple pusher 164. The knife 130 is disposed radially inward of the pair of annular arrays of staples 150. Accordingly, in use, as the staple pusher 164 is advanced, the knife 130 is also advanced axially outward.

A surgical buttress 124 is releasably attached to the staple cartridge 132 by the biodegradable adhesive (not shown). It is envisioned that the surgical buttress 124 may be additionally or alternatively attached to the anvil assembly 120. As illustrated in FIG. 6C, surgical buttress 124 is provided in an annular configuration and includes an aperture 129 that is sized and dimensioned to receive shaft 123 of anvil assembly 120 and allow free passage of knife 130 therethrough.

Referring again to FIG. 6B, surgical stapling apparatus 110 and detachable anvil assembly 120 are used in an anastomosis procedure to effect joining of intestinal sections. The anastomosis procedure is typically performed using minimally invasive surgical techniques including laparoscopic means and instrumentation. As shown in FIG. 6B, anvil assembly 120 has been applied to an operative site either through a surgical incision or transanally and positioned within a first intestinal section 50, and tubular body portion 114 of surgical stapling apparatus 110 has been inserted transanally into a second intestinal section 52.

Thereafter, the clinician maneuvers anvil assembly 120 until the proximal end of shaft 123 is inserted into the distal end of tubular body portion 114 of surgical stapling apparatus 110, wherein a mounting structure within the distal end of tubular body portion 114 engages shaft 123 to effect mounting. Anvil assembly 120 and tubular body portion 114 are then approximated to approximate intestinal sections 50 and 52. Surgical stapling apparatus 110 is then fired. The staples 150 are fired, effecting stapling of intestinal sections 50 and 52 to one another. The knife 130 cuts the tissue "T" to complete the anastomosis. Upon movement of anvil assembly 120 away from staple cartridge assembly 132, surgical buttress 124 is pulled away from staple cartridge assembly 132.

In embodiments, at least one bioactive agent may be combined with a surgical buttress of the present disclosure. The at least one bioactive agent may be disposed on a surface of the surgical buttress and/or impregnated therein. In these embodiments, the surgical buttress can also serve as a vehicle for delivery of the bioactive agent. The term "bioactive agent", as used herein, is used in its broadest sense and includes any substance or mixture of substances that have clinical use. Consequently, bioactive agents may or may not have pharmacological activity per se, e.g., a dye, or fragrance. Alternatively a bioactive agent could be any agent which provides a therapeutic or prophylactic effect, a compound that affects or participates in tissue growth, cell growth, cell differentiation, an anti-adhesive compound, a compound that may be able to invoke a biological action such as an immune response, or could play any other role in one or more biological processes. It is envisioned that the bioactive agent may be applied to the surgical buttress in any suitable form of matter, e.g., films, powders, liquids, gels and the like.

Examples of classes of bioactive agents which may be utilized in accordance with the present disclosure include anti-adhesives, antimicrobials, analgesics, antipyretics, anesthetics, antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, lipopolysaccharides, polysaccharides, and enzymes. It is also intended that combinations of bioactive agents may be used.

Other bioactive agents which may be included as a bioactive agent in the surgical buttress of the present disclosure include: local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents (e.g. oxybutynin); antitussives; bronchodilators; cardiovascular agents such as coronary vasodilators and nitroglycerin; alkaloids; analgesics; narcotics such as codeine, dihydrocodeinone, meperidine, morphine and the like; non-narcotics such as salicylates, aspirin, acetaminophen, d-propoxyphene and the like; opioid receptor antagonists, such as naltrexone and naloxone; anti-cancer agents; anti-convulsants; anti-emetics; antihistamines; anti-inflammatory agents such as hormonal agents, hydrocortisone, prednisolone, prednisone, non-hormonal agents, allopurinol, indomethacin, phenylbutazone and the like; prostaglandins and cytotoxic drugs; estrogens; antibacterials; antibiotics; anti-fungals; anti-virals; anticoagulants; anticonvulsants; antidepressants; antihistamines; and immunological agents.

Other examples of suitable bioactive agents which may be included include viruses and cells, peptides, polypeptides and proteins, analogs, muteins, and active fragments thereof, such as immunoglobulins, antibodies, cytokines (e.g. lymphokines, monokines, chemokines), blood clotting factors, hemopoietic factors, interleukins (IL-2, IL-3, IL-4, IL-6), interferons (β-IFN, (α-IFN and γ-IFN), erythropoietin, nucleases, tumor necrosis factor, colony stimulating factors (e.g., GCSF, GM-CSF, MCSF), insulin, anti-tumor agents and tumor suppressors, blood proteins, gonadotropins (e.g., FSH, LH, CG, etc.), hormones and hormone analogs (e.g., growth hormone), vaccines (e.g., tumoral, bacterial and viral antigens); somatostatin; antigens; blood coagulation factors; growth factors (e.g., nerve growth factor, insulin-like growth factor); protein inhibitors, protein antagonists, and protein agonists; nucleic acids, such as antisense molecules, DNA and RNA; oligonucleotides; polynucleotides; and ribozymes.

The examples below are illustrative polymer preparations for the adhesive of the present disclosure.

### EXAMPLE 1

A star polymer including about 75% by weight lactide and about 25% by weight caprolactone, and having a molecular weight from about 1,300 g/mol to about 2,000 g/mol, was prepared as follows:

In a dry room, about 6 grams of pentaerthritol, about 15.6 grams of caprolactone, about 58.67 grams of lactide, and about 0.0095 grams of stannous octoate were weighed and placed in a 100 ml two neck round bottom flask with mechanical stirrer. A pipette was inserted through a rubber septa above the level of the monomers and a syringe was used to pierce the rubber septa on the same arm. The mechanical stirrer was set at about 50 revolutions per minute (rpm) as nitrogen flowed over the monomers overnight.

The following day, the mixed components were heated with a hot plate/oil bath apparatus. The hot plate temperature was set to about 135°C with magnetic stirring of the oil bath at about 1000 rpm and the oil bath temperature at about 165°C, with polymer mixing in the flask at about 100 rpm. As the stannous octoate was low upon heating, an additional 0.003 grams was added, followed by another 0.003 grams, making the new target about 0.02% stannous octoate.

### EXAMPLE 2

A star polymer including about 75% by weight lactide and about 25% by weight caprolactone, and having a molecular weight from about 1,300 g/mol to about 2,000 g/mol, was prepared as follows:

In a dry room, about 5.5 grams of pentaerthritol, about 16.1 grams of caprolactone, about 67.3 grams of lactide, and about 0.0158 grams of stannous octoate were weighed and placed in a 100 ml two neck round bottom flask with mechanical stirrer. An pipette inlet was inserted through a rubber septa along with a needle outlet. Nitrogen was purged through the system, without heat, while mixing with the mechanical stirrer at about 100 rpm over a weekend.

The material was then moved to a microwave under static nitrogen. The microwave was set to ramp to a temperature of about 185°C over about 45 minutes. When observed at about 165°C, the solution was clear. With about 5 minutes left to ramp to about 185°C, the program was reset to a 30 minute ramp to about 185°C. After about 15 minutes at about 185°C, the solution was still clear. The temperature was increased to about 190°C where it was held for about 30 minutes, then to about 195°C for about 1 hour, and to about 200°C for about 40 minutes. Samples of the solution at 185°C and 195°C were compared by NMR scans, with very little change in caprolactone. The samples were then transferred into a PTFE dish and placed in a vacuum oven.

Persons skilled in the art will understand that the devices and methods specifically described herein and illustrated in the accompanying figures are non-limiting exemplary embodiments, and that the description, disclosure, and figures should be construed as merely exemplary of particular embodiments. It is to be understood, therefore, that the present disclosure is not limited to the precise embodiments described, and that various other changes and modifications may be effected by one skilled in the art without departing from the scope of the disclosure. Additionally, it is envisioned that the elements and features illustrated or described in connection with one exemplary embodiment may be combined with the elements and features of another exemplary embodiment without departing from the scope of the present disclosure, and that such modifications and variations are also intended to be included within the scope of the present disclosure. Accordingly, the subject matter of the present disclosure is not to be limited by what has been particularly shown and described, except as indicated by the appended claims.

## Claims

1. A surgical stapling apparatus including a releasable surgical buttress (24, 124), the surgical stapling apparatus comprising:
a cartridge assembly (32, 132) including a plurality of staples (50, 150) and a tissue contacting surface defining staple retaining slots;
an anvil assembly (20, 120) including a tissue contacting surface defining staple pockets for forming staples (50, 150) expelled from the staple retaining slots of the cartridge assembly (32, 132);
a surgical buttress (24, 124) disposed on at least one of the tissue contacting surfaces of the cartridge assembly (32, 132) and the anvil assembly (20, 120); and
a low molecular weight bioabsorbable adhesive for releasably retaining the surgical buttress (24, 124) on the at least one of the tissue contacting surfaces of the cartridge assembly (32, 132) and the anvil assembly (20, 120),
wherein the surgical buttress (24, 124) is porous and comprises a foam layer (23b),
the surgical buttress (24, 124) further comprising a further layer (23a) adhered to a surface of the foam layer (23b), wherein the further layer (23a) of the surgical buttress (24, 124) is adhered to the at least one of the tissue contacting surfaces of the cartridge assembly (32, 132) and the anvil assembly (20, 120) by the low molecular weight adhesive, and
the low molecular weight bioabsorbable adhesive having a low molecular weight of less than 3000 g/mol, **characterised in that** the further layer (23a) adhered to a surface of the foam layer (23b) is a non-woven fabric layer.

2. The surgical stapling apparatus according to claim 2, wherein the foam layer (23b) is fabricated from collagen.

3. The surgical stapling apparatus according to claim 2 or claim 3, preferably wherein the non-woven fabric layer (23a) is fabricated from an aliphatic polyester.

4. The surgical stapling apparatus of any preceding claim, further comprising a knife (30) disposed within a knife slot (25) formed in the tissue contacting surface of the cartridge assembly (32), wherein the surgical buttress (24) includes a gap (27) to allow free passage of the knife (30) through the knife slot (25).

5. The surgical stapling apparatus of any preceding claim, wherein the low molecular weight bioabsorbable adhesive has a molecular weight from 1,000 g/mol to 2,300 g/mol.

6. The surgical stapling apparatus according to claim 5 wherein the low molecular weight bioabsorbable adhesive has a molecular weight from 1,300 g/mol to 2,000 g/mol.

7. The surgical stapling apparatus of any preceding claim, wherein the low molecular weight bioabsorbable adhesive comprises an aliphatic polyester.

8. The surgical stapling apparatus according to claim 7 wherein the aliphatic polyester is a multi-arm star polymer.

9. The surgical stapling apparatus of claim 8, wherein the multi-arm star polymer comprises a copolymer of lactide and caprolactone.

10. The surgical stapling apparatus according to claim 9 wherein the copolymer includes about 75% by weight lactide and about 25% by weight caprolactone.

11. The surgical stapling apparatus of any preceding claim, wherein the cartridge assembly (32) is associated with a first jaw (22) and the anvil assembly is associated with a second jaw (20), the first and second jaws (20, 22) being selectively movable relative to one another from a first spaced apart position to a second position, wherein the first and second jaws (20, 22) cooperate to grasp tissue therebetween.

12. The surgical stapling apparatus of any of claims 1 to 10 , wherein the cartridge assembly (132) is associated with a body portion of the surgical stapling apparatus and the anvil assembly (120) includes a shaft removably mountable to the body portion, the anvil assembly (120) being movable toward and away from the body portion, and wherein the cartridge assembly (132) and the anvil assembly (120) are circular.

## Patentansprüche

1. Chirurgisches Klammergerät, das eine lösbare chirurgische Verstärkung (24, 124) umfasst, wobei das chirurgische Klammergerät umfasst:
eine Patronenanordnung (32, 132), die eine Mehrzahl von Klammern (50, 150) und eine Gewebekontaktfläche umfasst, welche Klammerrückhalteschlitze definiert;
eine Ambossanordnung (20, 120), die eine Gewebekontaktfläche umfasst, welche Klammertaschen zum Bilden von Klammern (50, 150) definiert, die aus den Klammerrückhalteschlitzen der Patronenanordnung (32, 132) ausgestoßen werden;
eine chirurgische Verstärkung (24, 124), die auf mindestens einer der Gewebekontaktflächen der Patronenanordnung (32, 132) und der Ambossanordnung (20, 120) angeordnet ist; und
einen biologisch resorbierbaren Klebstoff niedriger Molmasse zum lösbaren Halten der chirurgischen Verstärkung (24, 124) auf der mindestens einen der Gewebekontaktflächen der Patronenanordnung (32, 132) und der Ambossanordnung (20, 120),
wobei die chirurgische Verstärkung (24, 124) porös ist und eine Schaumstoffschicht (23b) umfasst,
die chirurgische Verstärkung (24, 124) ferner eine weitere Schicht (23a) umfasst, die an eine Oberfläche der Schaumstoffschicht (23b) geklebt ist, wobei die Schicht (23a) der chirurgischen Verstärkung (24, 124) durch den Klebstoff niedriger Molmasse an mindestens eine der Gewebekontaktflächen der Patronenanordnung (32, 132) und der Ambossanordnung (20, 120) geklebt ist, und
der biologisch resorbierbare Klebstoff niedriger Molmasse eine niedrige Molmasse von weniger als 3000 g/mol aufweist, **dadurch gekennzeichnet, dass** die weitere Schicht (23a), die an eine Oberfläche der Schaumstoffschicht (23b) geklebt ist, eine Vliesstoffschicht ist.

2. Chirurgisches Klammergerät nach Anspruch 2, wobei die Schaumstoffschicht (23b) aus Kollagen hergestellt ist.

3. Chirurgisches Klammergerät nach Anspruch 2 oder 3, wobei die Vliesstoffschicht (23a) vorzugsweise aus einem aliphatischen Polyester hergestellt ist.

4. Chirurgisches Klammergerät nach einem der vorhergehenden Ansprüche, ferner umfassend ein Messer (30), das innerhalb eines Messerschlitzes (25) angeordnet ist, der in der Gewebekontaktfläche der Patronenanordnung (32) ausgebildet ist, wobei die chirurgische Verstärkung (24) einen Spalt (27) umfasst, um den freien Durchtritt des Messers (30) durch den Messerschlitz (25) zu ermöglichen.

5. Chirurgisches Klammergerät nach einem der vorhergehenden Ansprüche, wobei der biologisch resorbierbare Klebstoff niedriger Molmasse eine Molmasse von 1.000 g/mol bis 2.300 g/mol aufweist.

6. Chirurgisches Klammergerät nach Anspruch 5, wobei der biologisch resorbierbare Klebstoff niedriger Molmasse eine Molmasse von 1.300 g/mol bis 2.000 g/mol aufweist.

7. Chirurgisches Klammergerät nach einem der vorhergehenden Ansprüche, wobei der biologisch resorbierbare Klebstoff niedriger Molmasse einen aliphatischen Polyester umfasst.

8. Chirurgisches Klammergerät nach Anspruch 7, wobei der aliphatische Polyester ein mehrarmiges Sternpolymer ist.

9. Chirurgisches Klammergerät nach Anspruch 8, wobei das mehrarmige Sternpolymer ein Copolymer von Lactid und Caprolacton umfasst.

10. Chirurgisches Klammergerät nach Anspruch 9, wobei das Copolymer etwa 75 Gewichts-% Lactid und etwa 25 Gewichts-% Caprolacton umfasst.

11. Chirurgisches Klammergerät nach einem der vorhergehenden Ansprüche, wobei die Patronenanordnung (32) mit einer ersten Backe (22) verbunden ist, und die Ambossanordnung mit einer zweiten Backe (20) verbunden ist, wobei die ersten und zweiten Backen (20, 22) in Bezug aufeinander selektiv aus einer ersten, beabstandeten Position in eine zweite Position bewegt werden können, wobei die ersten und zweiten Backen (20, 22) zusammenwirken, um Gewebe dazwischen zu ergreifen.

12. Chirurgisches Klammergerät nach einem der Ansprüche 1 bis 10, wobei die Patronenanordnung (132) mit einem Körperabschnitt des chirurgischen Klammergeräts verbunden ist, und die Ambossanordnung (120) einen Schaft umfasst, der abnehmbar am Körperabschnitt montiert werden kann, wobei die Ambossanordnung (120) in Richtung des Körperabschnitts und davon weg bewegt werden kann, und wobei die Patronenanordnung (132) und die Ambossanordnung (120) kreisförmig sind.

## Revendications

1. Appareil d'agrafage chirurgical comprenant un renfort chirurgical amovible (24, 124), l'appareil d'agrafage chirurgical comprenant :
un ensemble de cartouche (32, 132) comprenant une pluralité d'agrafes (50, 150) et une surface de contact avec le tissu définissant des fentes de retenue d'agrafe ;
un ensemble d'enclume (20, 120) comprenant une surface de contact avec le tissu définissant des poches d'agrafe pour former les agrafes (50, 150) expulsées des fentes de retenue d'agrafe de l'ensemble de cartouche (32, 132) ;
un renfort chirurgical (24, 124) disposé sur au moins l'une des surfaces de contact avec le tissu de l'ensemble de cartouche (32, 132) et de l'ensemble d'enclume (20, 120) ; et
une colle bioabsorbable à faible poids moléculaire pour retenir, de manière amovible, le renfort chirurgical (24, 124) sur au moins l'une des surfaces de contact avec le tissu de l'ensemble de cartouche (32, 132) et de l'ensemble d'enclume (20, 120),
dans lequel :
le renfort chirurgical (24, 124) est poreux et comprend une couche de mousse (23b),
le renfort chirurgical (24, 124) comprenant en outre une couche supplémentaire (23a) fixée sur une surface de la couche de mousse (23b), dans lequel la couche supplémentaire (23a) du renfort chirurgical (24, 124) est fixée sur au moins l'une des surfaces de contact avec le tissu de l'ensemble de cartouche (32, 132) et de l'ensemble d'enclume (20, 120) par la colle à faible poids moléculaire, et
la colle bioabsorbable à faible poids moléculaire ayant un faible poids moléculaire inférieur à 3000 g/mol, **caractérisé en ce que** la couche supplémentaire (23a) fixée sur une surface de la couche de mousse (23b) est une couche de tissu non tissé.

2. Appareil d'agrafage chirurgical selon la revendication 2, dans lequel la couche de mousse (23b) est fabriquée à partir de collagène.

3. Appareil d'agrafage chirurgical selon la revendication 2 ou la revendication 3, de préférence dans lequel la couche de tissu non tissé (23a) est fabriquée à partir d'un polyester aliphatique.

4. Appareil d'agrafage chirurgical selon l'une quelconque des revendications précédentes, comprenant en outre une lame (30) disposée à l'intérieur d'une fente de lame (25) formée dans la surface de contact avec le tissu de l'ensemble de cartouche (32), dans lequel le renfort chirurgical (24) comprend un espace (27) pour permettre le libre passage de la lame (30) à travers la fente de lame (25).

5. Appareil d'agrafage chirurgical selon l'une quelconque des revendications précédentes, dans lequel la colle bioabsorbable à faible poids moléculaire a un poids moléculaire de 1000 g/mol à 2300 g/mol.

6. Appareil d'agrafage chirurgical selon la revendication 5, dans lequel la colle bioabsorbable à faible poids moléculaire a un poids moléculaire de 1300 g/mol à 2000 g/mol.

7. Appareil d'agrafage chirurgical selon l'une quelconque des revendications précédentes, dans lequel la colle bioabsorbable à faible poids moléculaire comprend un polyester aliphatique.

8. Appareil d'agrafage chirurgical selon la revendication 7, dans lequel le polyester aliphatique est un polymère en étoile à plusieurs branches.

9. Appareil d'agrafage chirurgical selon la revendication 8, dans lequel le polymère en étoile à plusieurs branches comprend un copolymère de lactide et de caprolactone.

10. Appareil d'agrafage chirurgical selon la revendication 9, dans lequel le copolymère comprend environ 75% en poids de lactide et environ 25% en poids de caprolactone.

11. Appareil d'agrafage chirurgical selon l'une quelconque des revendications précédentes, dans lequel l'ensemble de cartouche (32) est associé à une première mâchoire (22) et l'ensemble d'enclume est associé à une seconde mâchoire (20), les première et seconde mâchoires (20, 22) étant sélectivement mobiles l'une par rapport à l'autre d'une première position espacée à une seconde position, dans laquelle les première et seconde mâchoires (20, 22) coopèrent pour saisir le tissu entre elles.

12. Appareil d'agrafage chirurgical selon l'une quelconque des revendications 1 à 10, dans lequel l'ensemble de cartouche (132) est associé à une partie de corps de l'appareil d'agrafage chirurgical et l'ensemble d'enclume (120) comprend une tige pouvant être montée de manière amovible sur la partie de corps, l'ensemble d'enclume (120) étant mobile vers et à distance de la partie de corps, et dans lequel l'ensemble de cartouche (132) et l'ensemble d'enclume (120) sont circulaires.
